# FASCICULE DE BREVET EUROPEEN

(11) **EP 0 880 650 B1**
(45) Date de publication et mention de la délivrance du brevet: **20.11.2002**
(21) Numéro de dépôt: 97905187.7
(22) Date de dépôt: 11.02.1997
(51) Int. Cl.: F04B 43/00, F04B 43/04

(54) **CIRCULATEUR DE FLUIDE A MEMBRANE VIBRANTE**
VIBRIERENDE MEMBRANPUMPE
FLUID CIRCULATOR WITH A VIBRATING MEMBRANE

(30) Priorité: 12.02.1996 FR 9601701
(43) Date de publication de la demande: 02.12.1998
(73) Titulaire: DREVET, Jean-Baptiste, F-75005 Paris (FR)
(72) Inventeur: DREVET, Jean-Baptiste, F-75005 Paris (FR)
(74) Mandataire: Robert, Jean-Pierre
(86) Numéro de dépôt international: FR9700262
(87) Numéro de publication internationale: WO97029282

(56) Documents cités:
- EP-A- 0 412 856
- WO-A-80/02445
- DE-A- 3 621 766
- SU-A- 244 126
- US-A- 4 488 854
- PATENT ABSTRACTS OF JAPAN vol. 015, no. 164 (M-1106), 24 Avril 1991 & JP 03 031590 A (HITACHI LTD), 12 Février 1991,
- PATENT ABSTRACTS OF JAPAN vol. 009, no. 132 (M-385), 7 Juin 1985 & JP 60 013994 A (KAETSU HOSHI), 24 Janvier 1985,
- PATENT ABSTRACTS OF JAPAN vol. 014, no. 496 (E-0996), 29 Octobre 1990 & JP 02 206369 A (AISIN SEIKI CO LTD), 16 Août 1990,
- PATENT ABSTRACTS OF JAPAN vol. 015, no. 253 (M-1129), 27 Juin 1991 & JP 03 081585 A (MITSUBISHI KASEI CORP), 5 Avril 1991,

## Description

La présente invention concerne un circulateur de fluide à membrane vibrante pour notamment fluides biologiques.

De nombreux types de pompes sont connus tant dans les domaines industriels que biomédicaux. On citera :
- les pompes volumétriques à cycle alternatif qui mettent en oeuvre comme éléments principaux, des pistons ou des membranes associées à des clapets d'admission et de refoulement. Leur principal inconvénient tient à l'aspect cyclique de leur mouvement et à la présence de clapets.
- les pompes volumétriques dites péristaltiques dans lesquelles des galets déforment et écrasent dans un mouvement continu un corps de pompe tubulaire souple. Cet écrasement peut être dommageable pour certains liquides à véhiculer qui comportent des éléments sensibles (sang).
- les pompes dites à impulseur telles que les pompes centrifuges à rotor et ailettes ou à vortex. Leur inconvénient tient à leur grande vitesse de rotation qui engendre un cisaillement des filets de fluide, des frictions, des cavitations, phénomènes dommageables pour des fluides fragiles.
- les pompes à turbine axiale dans lesquelles les inconvénients des pompes précédentes se retrouvent pour les fluides fragiles.

On connaît également un dispositif de propulsion de fluide décrit dans le document FR-A-2.650.862 qui met en oeuvre une membrane vibrante. Ce dispositif révèle une solution technique qui n'est pas toujours adaptée à l'obtention des performances hydrauliques requises par la plupart des applications industrielles et biomédicales.

Le circulateur de fluide à membrane vibrante selon l'invention propose des solutions par lesquelles les domaines d'application du circulateur sont plus vastes, les performances hydrauliques sont améliorées, l'encombrement du circulateur est réduit et enfin le corps de pompe peut être à usage unique ce qui est intéressant pour le domaine biomédical.

A cet effet le circulateur de fluide selon l'invention comprend un circuit hydraulique interne successivement composé d'un orifice d'admission, d'un corps de pompe et d'un orifice de refoulement, le corps de pompe délimitant en fonctionnement un espace à parois rigides entre lesquelles est placée une membrane déformable de propulsion, associée à des moyens de création d'une tension parallèle à la circulation du fluide, pour constituer le support d'ondes se déplaçant de l'extrémité soumise à la force d'excitation vers son autre extrémité située du côté de l'orifice de refoulement, et comportant des moyens pour son attelage par son extrémité du côté de l'orifice d'admission à un organe moteur engendrant une force d'excitation périodique sensiblement normale à sa surface, conformément au document FR 2650862.

Selon l'invention, l'écartement des parois rigides est décroissant depuis l'orifice d'admission jusqu'à l'orifice de refoulement pour créer un amortissement forcé des ondes au cours de leur déplacement le long de la membrane, afin de créer un transfert d'énergie entre la membrane et le fluide se traduisant sous la forme d'un gradient de pression et d'un débit de fluide.

Les caractéristiques de ce gradient de pression et de ce débit sont en relation avec les dimensions du corps de pompe et celles de la membrane, la forme et l'écartement des parois rigides, les caractéristiques mécaniques et l'état de tension de la membrane et les paramètres de son excitation.

Selon un mode de réalisation préféré, l'excitation périodique de la membrane est réalisée à l'une des fréquences propres de celle-ci, notamment la première fréquence propre. La valeur de ces fréquences propres est liée aux caractéristiques mécaniques de la membrane et à son état de tension. Cette fréquence d'excitation sera limitée à des valeurs faibles de l'ordre de 40 à 50 Herts de manière à éviter des effets de pression localisée et des effets de cisaillement des filets de fluide.

Dans un autre mode de réalisation, l'espace à parois rigides est délimité par deux parois de forme discoïdale entre lesquelles est placée une membrane déformable de forme également discoïdale. Cette architecture circulaire possède un effet de concentration de l'énergie rayonnant de l'extérieur vers le centre du système et permet d'engendrer des gradients de pression compatibles avec ceux exigés par des applications industrielles et biomédicales. Cette solution permet aussi un fonctionnement avec des très faibles amplitudes d'excitation en périphérie, ce qui permet d'éviter le traumatisme de fluides fragiles. Elle présente aussi l'avantage de permettre une mise en tension très simple de la membrane puisqu'il suffit d'agir sur son bord externe uniquement.

Dans un autre mode de réalisation, la membrane est constituée par l'assemblage de membranes superposées, celles-ci étant séparées par de fines entretoises d'une matière de très faible raideur. Cette disposition permet la réalisation d'une membrane de masse accrue et de fréquence propre voisine de la fréquence propre de chaque membrane dans le cas de l'assemblage de membranes identiques.

Dans un autre mode de réalisation des déflecteurs souples sont portés par la membrane et s'appuient sur le corps de pompes. Ces déflecteurs modifient la longueur du circuit hydraulique et l'évolution des vitesses d'écoulement du fluide dans l'espace de circulation. Cette disposition permet au circulateur de fonctionner avec un écartement des parois rigides plus important ce qui est favorable à la propulsion de fluides fragiles ou chargés de particules.

Selon un autre mode de réalisation, des lèvres souples circulaires sont portées par la membrane et s'appuient sur le corps de pompe, ces lèvres offrent ainsi une solution simple de valve anti-retour vis-à-vis du débit de fluide pour satisfaire des exigences requises notamment dans certaines applications biomédicales.

Dans un autre mode de réalisation, le corps de pompe du circulateur forme une unité intégrant de manière inséparable la membrane, les orifices d'admission et de refoulement, et les parois délimitant l'espace dans lequel est logée la membrane. Ces parois sont rigides et maintenues écartées l'une de l'autre par des moyens d'entretoisement. Elles sont reliées en périphérie à la membrane par des parois souples. Cette membrane comporte, à l'intérieur de l'espace de circulation, des perforations à cette périphérie et un orifice central qui permettent la circulation du fluide de part et d'autre de la membrane. Cette disposition technique permet de réaliser un corps de pompe dont le fonctionnement n'est pas perturbé par la valeur de la pression d'admission.

Pour ce qui concerne les moyens d'excitation de la membrane, ceux-ci sont constitués par un moteur électromagnétique dont le circuit d'alimentation en courant alternatif d'excitation comporte un circuit amplificateur de puissance et un circuit générateur du signal d'excitation de manière à offrir des possibilités de modulation d'amplitude, de programmation, de mémorisation et de génération de signaux d'excitation complexes, simulant par exemple les pulsations cardiaques.

D'autres caractéristiques et avantages ressortiront de la description donnée ci-après de divers modes de réalisation de l'invention.

Il sera fait référence aux dessins annexés dans lesquels :
- la figure 1 est une vue en coupe longitudinale d'un corps de pompe tubulaire pour circulateur de fluide de type longitudinal, cette vue étant partielle et schématique;
- la figure 2 est une vue en coupe longitudinale d'un corps de pompe de circulateur de fluide de type cylindrique ;
- la figure 3 est une vue en coupe diamétrale partielle et schématique du corps de pompe d'un circulateur de fluide de type circulaire ;
- la figure 4 est une vue d'ensemble en coupe diamétrale d'un premier mode de réalisation du dispositif selon la figure 3 ;
- les figures 4a, 4b et 4c sont trois courbes en fonction du temps représentant respectivement l'amplitude du signal d'excitation, l'amplitude du courant d'excitation du moteur et le gradient de pression hydraulique d'une pompe en fonctionnement.
- les figures 5 à 10 sont des demi-coupes radiales de différentes membranes susceptibles d'être mises en oeuvre dans le circulateur de l'invention,
- les figures 11 et 12, respectivement en plan et en perspective concernent une membrane comportant des déflecteurs,
- la figure 13 illustre par une demi-coupe radiale une membrane à reliefs concentriques logée dans son espace de circulation,
- la figure 14 est un schéma d'une variante de réalisation de la membrane dans laquelle le circuit hydraulique est limité par rapport à la surface de la membrane,
- la figure 15 illustre une membrane comportant des lèvres anti-retour logées dans son espace de circulation;
- la figure 16 illustre par une demi-coupe radiale un espace de circulation équipé d'une membrane dans lequel les parois de l'espace comportent des lèvres anti-retour;
- les figures 17 et 18 illustrent une variante de réalisation du circulateur dans lequel la membrane comporte des éléments de liaison souples avec le corps de pompe;
- les figures 19 à 27 sont des vues en coupe de plusieurs modes de réalisation de corps de pompe à membrane incorporée, les rendant ainsi à usage unique ou démontables;
- les figures 28 à 30 illustrent la liaison entre la membrane et un organe d'excitation, dans les corps de pompe représentés aux figures 21 et 22;
- la figure 31 illustre un autre mode de liaison entre la membrane et l'organe d'excitation dans un corps de pompe du type de celui de la figure 24;
- la figure 32 illustre par une demi-coupe radiale une membrane comportant un élément sensible à un champ magnétique engendré par l'organe d'excitation ;
- la figure 33 illustre par une demi-coupe radiale un corps de pompe comportant un élément moteur d'excitation.

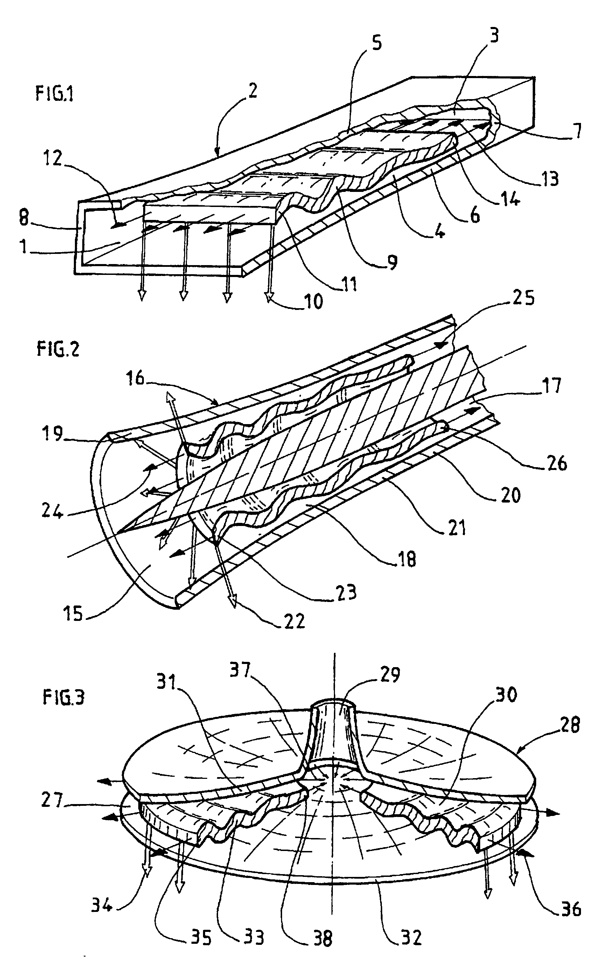

Le dispositif de l'invention représenté à la figure 1 comporte un circuit hydraulique composé successivement d'un orifice d'admission 1, d'un corps de pompe 2 et d'un orifice de refoulement 3. Le corps de pompe 2 est un tube plat de section évolutive qui délimite l'espace 4 de circulation par des parois rigides 5, 6, 7 et 8. Dans l'espace 4 est logée une membrane déformable de propulsion 9 qui se présente comme une lame souple d'élastomère de largeur égale à l'écartement entre les parois 7 et 8. Un organe moteur non représenté engendre une force d'excitation périodique 10 qui est appliquée au moyen d'un attelage à l'extrémité 11 de cette membrane 9 du côté de l'orifice d'admission 1, cette force étant régulièrement répartie sur le bord de la membrane et ayant une direction normale à celle-ci. Cette membrane 9 est maintenue en tension par des organes non représentés, développant des forces 12 et 13 de sens opposés et appliquées aux extrémités 11 et 14 de la membrane. La membrane, lorsqu'elle est excitée, est alors le support d'ondes concentriques, se déplaçant de l'extrémité 11 soumise à l'excitation vers l'autre extrémité 14 située du côté de l'orifice de refoulement. Le déplacement des ondes s'accompagne d'un amortissement forcé, dû à la forme et à l'écartement des parois rigides 5 et 6, cet écartement étant de préférence décroissant dans son évolution depuis l'orifice d'admission jusqu'à l'orifice de refoulement.

Cet amortissement se traduit par un transfert d'énergie entre la membrane 9 et le fluide, ceci sous la forme d'un gradient de pression et d'un débit de fluide.

Globalement ce circulateur se présente comme un transducteur d'énergie, engendrant successivement un transfert d'énergie de l'organe moteur d'excitation à la membrane, puis de la membrane au fluide. L'énergie fournie par l'excitateur dépend de plusieurs paramètres tels que la force d'excitation, la fréquence d'excitation qui sera de préférence la première fréquence propre de la membrane, l'amplitude d'excitation, celle-ci étant elle-même liée à la fréquence et à la force d'excitation. Il est ainsi possible de moduler l'énergie fournie par l'excitateur en agissant sur les différents paramètres qui régissent l'énergie fournie à la membrane.

L'énergie mécanique dans la membrane 9 doit se comprendre essentiellement comme un flux d'énergie mécanique se propageant au moyen de cette membrane depuis le point d'excitation 11 lieu du transfert d'énergie de l'excitateur à la membrane vers l'autre extrémité 14 de la membrane. Cette énergie comportant une partie d'énergie cinétique et une partie d'énergie de déformation, des limites physiques s'imposent à ce fonctionnement. Le transfert d'énergie de la membrane au fluide se réalise progressivement sur la longueur de la membrane avec la propagation et l'amortissement simultanés des ondes.

L'énergie hydraulique du fluide s'exprime par la puissance hydraulique fournie par le circulateur, c'est-à-dire le produit entre le débit et le gradient de pression, la relation entre le débit et la pression étant dépendante principalement des dimensions du corps de pompe et de la membrane, de l'écartement et de la forme des parois rigides 5 et 6, ceci en tenant compte des pertes de charges internes au système.

Une variante du dispositif est représentée à la figure 2, dans laquelle le circuit hydraulique est cylindrique et se compose d'un orifice d'admission 15, d'un corps de pompe 16 et d'un orifice de refoulement 17, le corps de pompe 16 délimitant l'espace de circulation 18 par des parois 19 et 20 rigides de révolution et coaxiales. Une membrane tubulaire déformable 21 est logée dans l'espace 18 tubulaire et est réalisée par exemple en élastomère silicone. Un organe moteur d'excitation non représenté engendre une distribution radiale et symétrique de forces d'excitation périodique 22, cette distribution de forces étant appliquée au moyen d'un attelage à l'extrémité 23 de la membrane tubulaire 21 du côté de l'orifice d'admission. Cette membrane est maintenue en tension axiale entre l'admission et le refoulement par des moyens non représentés engendrant une distribution axiale de forces de tension 24, 25 de sens opposés et appliquée aux extrémités 23 et 26 de la membrane.

A la figure 3 on a représenté une variante circulaire du dispositif selon l'invention où le circuit hydraulique se compose de l'orifice d'admission 27, du corps de pompe 28 et de l'orifice de refoulement 29, le corps de pompe 28 délimitant un espace de circulation 30 par ses parois rigides 31 et 32. Dans l'espace 30 en forme de disque et d'épaisseur décroissante, une membrane déformable également en forme de disque 33 est logée. Elle est réalisée par exemple en élastomère silicone.

Un organe moteur d'excitation engendre une distribution cylindrique et symétrique de forces d'excitation périodique 34, cette distribution de forces étant appliquée au moyen d'un attelage à l'extrémité périphérique 35 de la membrane 33 du côté de l'orifice d'admission. Cette membrane est en outre maintenue en tension par des moyens non représentés engendrant une distribution de forces radiales 36 et 37 en sens opposés appliquée à l'extrémité périphérique 35 et à l'extrémité centrale 38 de la membrane 33. On notera que les forces de tension résultent de la géométrie même de la membrane à laquelle on applique des forces divergentes sur son bord extérieur 35.

La membrane devient donc le support d'ondes concentriques qui se déplacent depuis son bord 35 vers son centre, ce déplacement s'accompagnant comme exposé précédemment de l'amortissement des ondes et de la propulsion du fluide. Un effet particulier à cette variante réside dans la concentration de l'énergie qui se propage de la périphérie vers le centre ceci permettant d'engendrer des gradients de pression plus importants que pour les variantes de circulateur décrites précédemment.

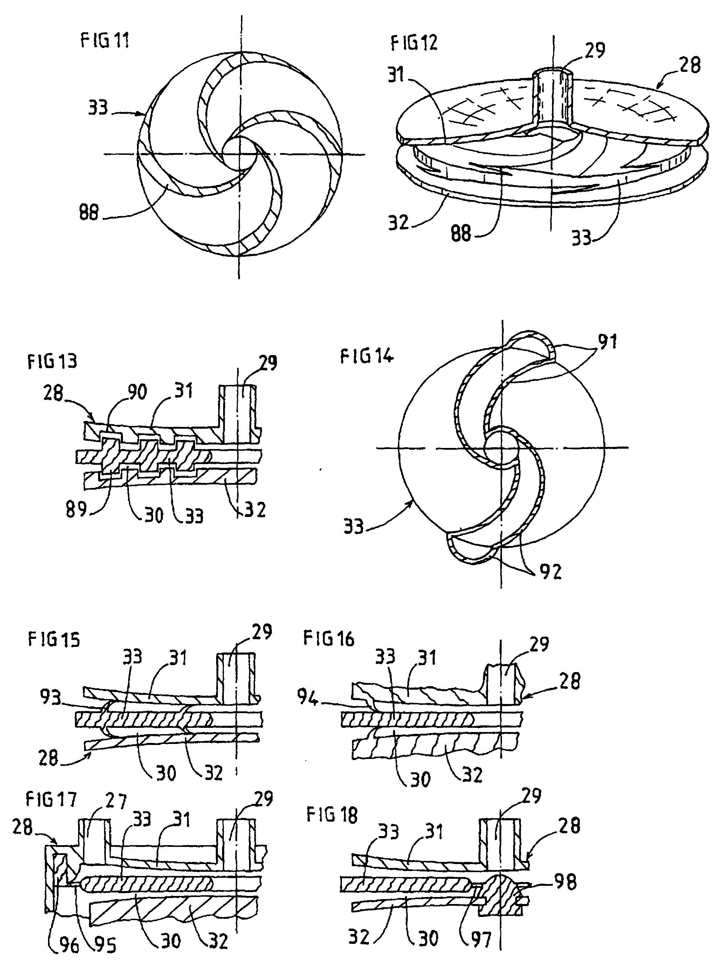

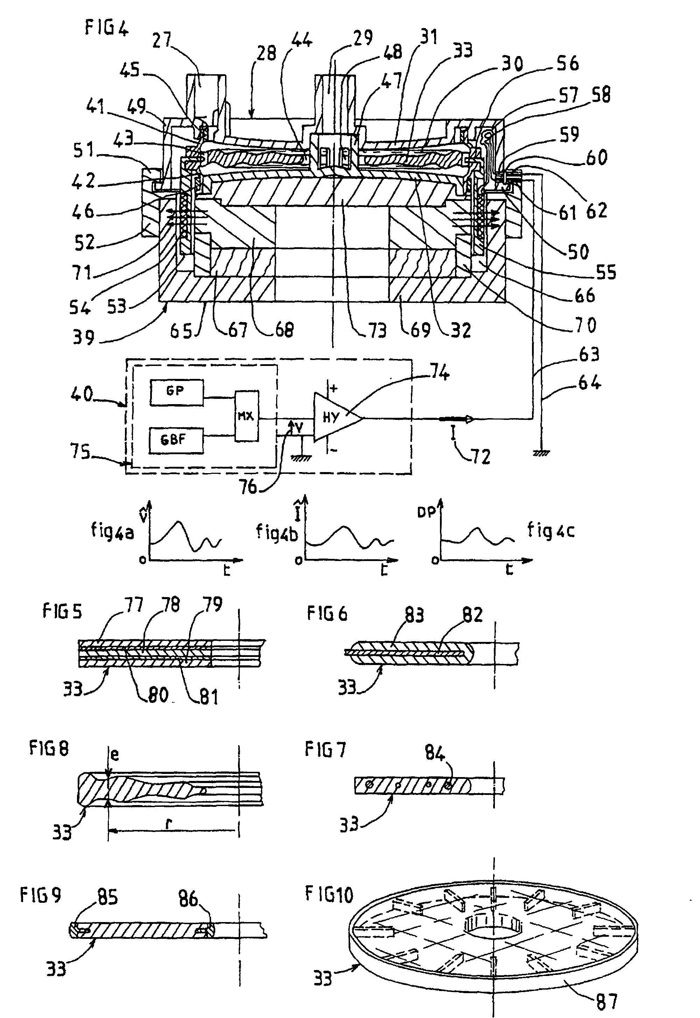

La figure 4 est une vue d'ensemble du circulateur circulaire représenté à la figure 3. Ce circulateur comprend trois sous-ensembles principaux, un corps de pompe 28 à usage unique ou démontable, un moteur d'excitation 39 fixé sur son bâti et une alimentation électrique 40.

Le corps de pompe 28 délimite l'espace 30 de propulsion du fluide en forme de disque où se situe la membrane déformable vibrante 33. Cet espace 30 est limité d'une part par des parois rigides supérieure et inférieure 31, 32, d'autre part par des parois souples 41 et 42 qui relient en périphérie la membrane 33 aux parois 31, 32. La membrane 33 et les parois souples 41 et 42 forment une pièce unique moulée en élastomère silicone, polyuréthane, caoutchouc ou analogue.

A l'intérieur de l'espace 30 la membrane 33 comporte des perforations 43 en périphérie et un orifice central 44 qui permettent au fluide de circuler de part et d'autre de la membrane 33. Les bords des parois souples 41, 42 sont fixées aux parois rigides 31, 32 par collage et pincement au moyen de bagues 45, 46 par exemple en acier inoxydable. Les parois 31 et 32 sont reliées entre elles, centrées et maintenues au bon écartement par une entretoise 47 portée par la paroi supérieure 31, l'assemblage étant réalisé par collage ou soudage aux ultrasons. Cette entretoise 47 comporte des orifices 48 pour le passage du fluide dans l'orifice de refoulement. La paroi supérieure 31 supporte en son centre l'orifice de refoulement 29 et à sa périphérie l'orifice d'admission 27. Elle comporte également une jupe extérieure 49 formant protection des côtés du corps de pompe 28. Cette jupe se termine par des portions de bourrelet 50 qui coopèrent avec des portions de collerette 51 d'une bague de liaison 52 solidaire du moteur 39. La fixation du corps de pompe au moteur est une fixation à baïonnette. Les parois rigides 31, 32 sont moulées en plastique rigide biocompatible du genre polysulfone.

Dans cette variante du corps de pompe, la membrane 33 comporte à sa périphérie une bobine mobile 53, élément mobile du moteur d'excitation 39. Cette bobine mobile 53 comporte un enroulement de fils conducteurs isolés 54 monté sur un support de bobine 55 rigide, mince et cylindrique. Ce support de bobine 55 comporte à son bord supérieur une collerette rigide 56 qui, perforée, pénètre dans la membrane 33 pour sa fixation par collage ou par surmoulage. Les fils d'alimentation 57, 58 relient l'enroulement 54 aux connecteurs 59, 60 portés par la jupe 49, ces connecteurs 59, 60 étant au contact des connecteurs 61, 62 portés par la bague de liaison 52 et eux-mêmes reliés à l'alimentation électrique du circulateur par des fils 63 et 64. Le support de bobine 55 est réalisé dans un matériau plastique à forte résistance mécanique et à grande stabilité dimensionnelle du genre polycarbonate chargé de fibre de verre.

Le moteur 39 se compose de la bobine mobile 53 décrite ci-dessus et d'un circuit magnétique 65 fixe qui comporte un entrefer 66 dans lequel évolue l'enroulement 54 de la bobine 53. Le circuit magnétique 65 comporte un aimant permanent 67, une armature intérieure 68 et une armature extérieure 69 qui encadrent l'aimant permanent 67 et qui sont centrées l'une par rapport à l'autre par une entretoise 70. L'espace entre ces deux armatures forme l'entrefer 66 où peut se déplacer l'enroulement 54 et où règne un champ magnétique 71 fixe permanent et de direction radiale. La distribution des forces d'excitation 34 (voir figure 3) résulte des forces de Laplace qui s'appliquent à la portion d'enroulement 54 parcourue par un courant alternatif d'excitation 72 et plongée dans le champ magnétique d'entrefer 71.

Un couvercle 73 permet le centrage et le bon positionnement du corps de pompe 28 sur le moteur d'excitation 39. Les armatures 68 et 69 sont à forte perméabilité magnétique par exemple en ferrite douce ou en acier doux. L'aimant permanent 67 est réalisé dans une matière magnétique de type terres rares, ferrite dure... On notera que l'aimant et les armatures peuvent être obtenus par moulage d'une matière composite comportant une matrice en matière plastique à l'intérieur de laquelle est dispersée une poudre de matière magnétique. L'entretoise 70, le couvercle 73 et la bague 52 sont en matière amagnétique.

Le circuit d'alimentation 40 fournissant le courant d'excitation 72 à l'enroulement 54 comporte un amplificateur de puissance 74 et un générateur 75 de signal. L'amplificateur de puissance 74 est un circuit hybride, standard, de type audio, dont les caractéristiques sont bien adaptées à cette application. Le générateur 75 qui alimente l'amplificateur 74 comporte essentiellement trois éléments : un oscillateur, un modulateur, et un mélangeur. L'oscillateur engendre un signal alternatif de base, fixe en amplitude et en fréquence, qui est la fréquence propre de la membrane 33. Le modulateur possède des capacités de programmation et de mémorisation de données ; il engendre le signal périodique de modulation de l'amplitude du signal de base. Le circuit mélangeur module le signal de base par le signal de modulation pour délivrer un signal d'excitation 76. Le mode de réalisation de l'alimentation électrique du circulateur permet un fonctionnement tel qu'illustré par les courbes en fonction du temps selon les figures 4a, 4b, 4c. La figure 4a est un graphe de l'amplitude du signal d'excitation 76, la figure 4b représente l'amplitude du courant d'excitation 72 et la figure 4c le gradient de pression généré par le circulateur. Il est ainsi possible d'engendrer des cycles de pression pulsée reproduisant les cycles de pulsations cardiaques pour une application biomédicale en circulation extra-corporelle du sang.

La membrane 33 présentée à la figure 5 est une membrane composite formée par l'assemblage de trois membranes semblables 77, 78, 79 superposées et séparées par deux entretoises fines 80 et 81 de très faible raideur. L'assemblage est réalisé par collage, les membranes sont en élastomère et les entretoises en mousse d'élastomère. Cette disposition permet de réaliser une membrane de fréquence propre voisine de la fréquence propre de chaque membrane mais avec une masse trois fois plus importante que la masse individuelle d'une membrane.

A la figure 6 la membrane 33 comporte une âme 82 de raideur déterminée recouverte par une épaisseur 83 d'une autre matière et de raideur plus faible que celle de l'âme 82. Par exemple, l'âme est réalisée dans un élastomère de forte dureté (80 shores A) tandis que la couche 83 est un surmoulage de gel d'élastomère de dureté shore A de l'ordre de 20 (silicone ou polyuréthane).

Dans cette disposition, la membrane à une raideur proche de celle de l'âme et une masse nettement accrue par rapport à celle de l'âme, ceci permet d'obtenir une fréquence propre plus faible que celle de l'âme seule.

Dans la membrane 33 de la figure 7, on a placé des inclusions 84 dans un élastomère silicone ou polyuréthane. L'inclusion peut être de forte ou de faible densité. Cette disposition permet de moduler la répartition des masses dans la membrane 33 et ainsi de modifier les modes de vibration et le profil des amplitudes de cette vibration. Ce mode de réalisation permet de laisser une grande liberté de dessin du profil extérieur de la membrane pour qu'il puisse s'adapter au paramètre d'écoulement du fluide.

Le schéma de la figure 8 illustre une membrane de propulsion 33 qui est homogène mais dont l'épaisseur e est évolutive en fonction du rayon r et de son emplacement angulaire, ceci dans le but de moduler la raideur et de répartir les masses afin d'améliorer et d'optimiser les caractéristiques dynamiques de cette membrane.

A la figure 9 la membrane représentée 33 est en élastomère et comporte à ses extrémités 35, 38 en périphérie et au centre, des pièces rigides circulaires 85 et 86 assemblées par collage ou surmoulage. Ces pièces augmentent la raideur de la membrane et surtout constituent des éléments créateurs de tensions internes radiales dans la membrane qui sont liées aux contraintes de déformation. On peut ainsi modifier les fréquences propres de cette membrane sans en accroître la masse. Les pièces 85 et 86 sont réalisées dans un plastique rigide biocompatible du genre polysulfone. On notera que le rôle de la pièce 85 est joué par le support rigide 55 de la figure 4.

La figure 10 décrit une autre variante de la membrane 33 dans laquelle est intégrée une structure 87 en plastique rigide par un surmoulage partiel. Cette structure 87 permet de modifier la raideur de la membrane en élastomère et peut coopérer à l'attelage de cette membrane à l'organe moteur d'excitation 39.

Les exemples ci-dessus décrits ne sont pas limitatifs pour ajuster la raideur de la membrane : on peut en effet agir sur la composition des matériaux qui constituent cette membrane ou intégrer à un matériau donné d'autres types de structures rigidifiantes ou enfin conformer la membrane pour qu'elle comporte par exemple des nervures.

Les figures 11 et 12 représentent une variante particulière de la membrane 33, celle-ci portant des déflecteurs souples 88 qui s'appuient sur les parois rigides 31 et 32 du corps de pompe. La membrane 33 et ses déflecteurs 88 sont en une pièce unique formée par moulage en élastomère. Ces déflecteurs 88 augmentent la longueur du circuit hydraulique et modifient l'évolution des vitesses d'écoulement du fluide dans l'espace de circulation 30. On peut ainsi construire un circulateur, où l'écartement des parois 31 et 32 est important, ce qui est recherché pour des applications de propulsion de fluide chargé tel que les pompes pour lave-linge et les pompes de lave-vaisselle.

La figure 13 décrit une autre disposition technique permettant d'agir sur les paramètres de l'écoulement dans l'espace de circulation 30. Des bourrelets concentriques 89 portés par la membrane 33 et des rainures creuses concentriques 90 ménagées dans les parois 31 et 32 coopèrent pour allonger le circuit hydraulique et ajuster en la réduisant la section de ce circuit dans l'espace 30. Ce mode de réalisation s'applique bien à un type de pompe de faible débit et de gradient de pression élevé tel que les micropompes électroménagères pour fer à vapeur ou les pompes de lave-glaces d'automobiles.

A la figure 14, l'espace de circulation 30 est délimité par des parois souples 91, 92 qui limitent et réduisent la surface de cet espace vis-à-vis de la surface de la membrane 33. On accroît ainsi le gradient de pression engendré par le circulateur et ces pompes sont applicables à des conditions d'utilisation demandant de faibles débits et de forts gradients de pression. La membrane 33 et les parois souples 91, 92 forment une pièce unique moulée en élastomère.

Aux figures 15 et 16, le circuit hydraulique comporte des valves anti-retour pour le fluide. Ces valves sont réalisées de manière simple au moyen de lèvres souples et circulaires disposées dans l'espace de circulation 30 entre les parois fixes et la membrane. A la figure 15 ces lèvres 93 sont portées par la membrane 33 avec laquelle elles forment une pièce unique, moulée en élastomère tandis qu'à la figure 16 ces lèvres souples sont portées par les parois 31 et 32. Ce type de réalisation trouve une application dans certaines pompes biomédicales.

Les déflecteurs 88, bourrelets 89, parois 91, 92 ou lèvres 93, 94 des figures 12 à 16 peuvent être réalisés par des éléments distincts de la membrane et du corps de pompe et maintenus par des moyens de calage et des formes appropriées entre eux dans l'espace de circulation.

La membrane 33 est, aux figures 17 et 18, reliée au corps de pompe 28 de manière souple par un voile qui la prolonge et qui aboutit à une pièce de fixation encastrée et collée dans le corps de pompe 28. Ce voile comporte des perforations permettant la circulation du fluide et est en une seule pièce avec la membrane et la pièce de fixation, par exemple moulée en élastomère. Cette liaison assure le centrage de la membrane dans le corps de pompe et limite l'amplitude de battement de l'extrémité de la membrane prolongée par le voile, notamment dans une phase d'amorçage du circulateur. A la figure 17, le voile 95 prolonge la membrane au-delà de son bord extérieur et aboutit à une pièce circulaire encastrée en périphérie dans la paroi 31 tandis qu'à la figure 18 le voile 97 prolonge l'extrémité interne de la membrane et aboutit à un plot central 98 encastré dans la paroi 32.

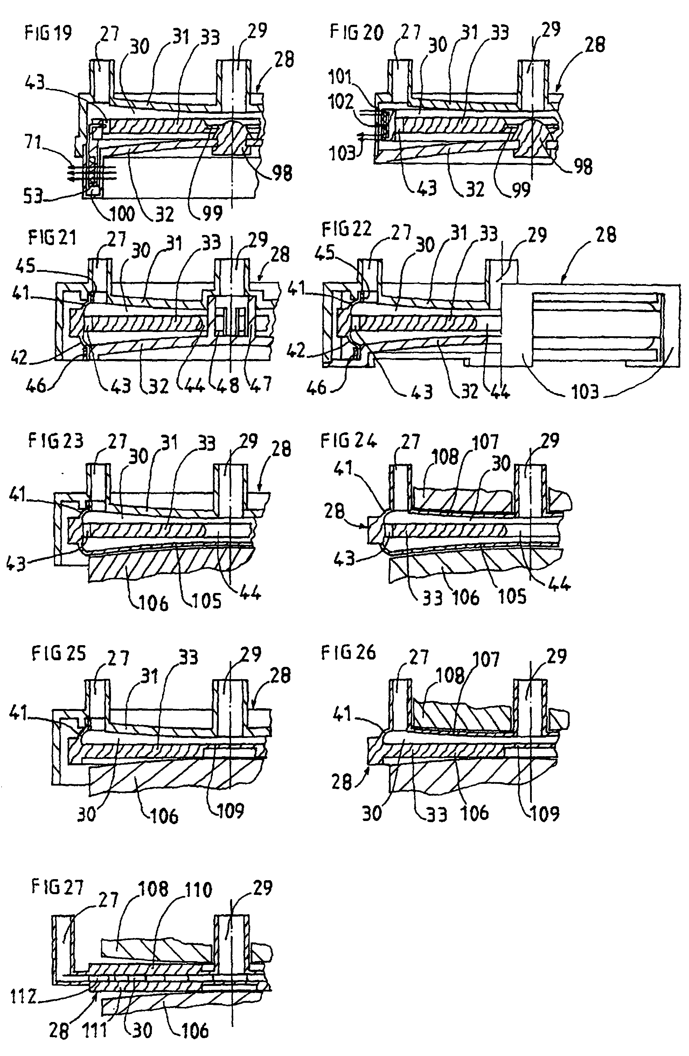

Les figures 19 et 20 illustrent deux variantes de réalisation de corps de pompe à usage unique d'un circulateur de type circulaire. Ces variantes sont du type à boîtier rigide et fermé.

C'est ainsi que les parois rigides 31 et 32 sont les parois supérieure et inférieure d'un boîtier fermé ; elles délimitent entre elles l'espace de circulation 30, ce boîtier étant équipé des orifices d'admission 27 et de refoulement 29 au niveau de sa paroi 31 et renfermant la membrane 33 de propulsion à l'intérieur de l'espace de circulation 30. Le centrage de la membrane par rapport au boîtier est dans ces figures assuré comme à la figure 18 au moyen d'un plot 98 relié à l'extrémité intérieure de la membrane par un voile 97 qui comporte des perforations 99. De même qu'à la figure 4, la membrane possède également des perforations périphériques 43, ces perforations permettant la circulation du fluide. Dans le mode de réalisation de la figure 19, le boîtier comporte un logement annulaire 100 de faible largeur à paroi fine et rigide logeant une bobine mobile d'excitation 53 solidaire de la membrane 33 à l'instar de la figure 4. En cours de fonctionnement, ce logement 100 est placé dans l'entrefer du circuit magnétique d'un moteur d'excitation, le champs magnétique 71 d'entrefer traversant ce logement 100. A la figure 18, le boîtier est réalisé sous une autre forme : il comporte à sa périphérie une paroi fine et rigide 101 en regard d'une armature 102 en matière magnétique du type ferrite douce solidaire de la membrane 33. En fonctionnement cette paroi 101 est placée dans l'entrefer d'un circuit magnétique multipolaire passant par l'armature 102, le champs magnétique 103 du moteur entrant et sortant par cette même paroi 101.

Les figures 21 et 22 illustrent deux autres variantes de réalisation d'un corps de pompe à usage unique, c'est-à-dire jetable. La différence de cette réalisation par rapport à celle des deux figures précédentes réside dans le fait qu'elle permet grâce à des parois souples 41 et 42, qui relient en périphérie la membrane 33 avec les parois rigides 31 et 32, de limiter l'espace de circulation 30, indépendamment de la structure rigide qui porte les parois 31 et 32. Cela permet alors comme à la figure 4 de réaliser un accouplement direct de la membrane à l'armature mobile d'un moteur d'excitation grâce à la partie de membrane qui fait saillie à l'extérieur des parois 41 et 42. Le corps de pompe illustré à la figure 21 est pour sa partie centrale semblable à celui illustré par la figure 4. En revanche, la réalisation illustrée par la figure 22 comporte une liaison des deux parois rigides 31, 32 au moyen de quatre entretoises 103 qui les relient en périphérie. Les espaces 104 entre ces entretoises permettent la mise en place des moyens d'attelage de la membrane à l'organe moteur d'excitation.

La variante de réalisation du corps de pompe illustré par la figure 23 se distingue de celle de la figure 22 par le fait que l'espace de circulation n'est pas directement limité par une paroi rigide telle que 32, mais par une paroi souple 105 qui prolonge le voile 42 et ferme en partie inférieure l'espace de circulation. Dans ce cas, le bâti du moteur comporte une paroi rigide 106 sur laquelle vient reposer la paroi souple 105 lorsque le corps de pompe est attelé au moteur d'excitation 39. Cette paroi rigide 106 n'est pas jetable.

A la figure 24 une autre variante est représentée, dans le même esprit que celle de la figure 23, c'est-à-dire que le corps de pompe qui intègre la membrane est limité par deux parois souples 105 et 107 qui, lorsqu'elles sont en service, coopèrent avec deux parois rigides 106 et 108 de la pompe afin de délimiter l'espace de circulation. Dans ce cas la paroi souple supérieure 107 est en une seule pièce d'une part avec la paroi souple 105 et la membrane 33 et d'autre part avec les embouts 27 et 29 constituant les orifices d'admission et d'échappement de l'espace de circulation du fluide. En service, on insère le corps de pompe entre les parois rigides 106 et 108 qui appartiennent au bâti du circulateur et qui ne sont pas jetables, et on attèle la membrane 33 au moteur d'excitation 39.

Les variantes de réalisation illustrées par les figures 25 et 26 pour le corps de pompe diffèrent respectivement des variantes 23 et 24 par l'absence de la paroi 105. En d'autres termes, le fluide situé dans l'espace de circulation 30 ne coopère qu'avec une seule face de la membrane 33, celle située à l'opposé de celle située en regard de la pièce 106. Dans ce cas la membrane est dépourvue d'orifices 43 et 44, un voile 109 souple refermant l'orifice central 44. Les deux corps de pompe de ces figures 25 et 26 se montent de la même manière que ceux respectivement des figures 23 et 24.

La figure 27, dans le même esprit que les variantes des figures 25 et 26, illustre une variante dans laquelle l'espace de circulation se trouve limité entre deux membranes 110 et 111 écartées l'une de l'autre par des entretoises 112 et placées en service entre les pièces fixes 106 et 108 dont le profil est identique à celui des parois fixes 31 et 32 précédemment décrites. L'espace de circulation 30 est connecté à un orifice d'admission 27 et ce de manière souple ainsi qu'à un orifice d'évacuation 29, les embouts qui matérialisent ces orifices étant en une seule pièce avec les deux membranes entretoisées, l'ensemble constituant le corps de pompe à usage unique et jetable. On notera que dans ce mode de réalisation, un bon fonctionnement demande que la pression d'admission soit positive.

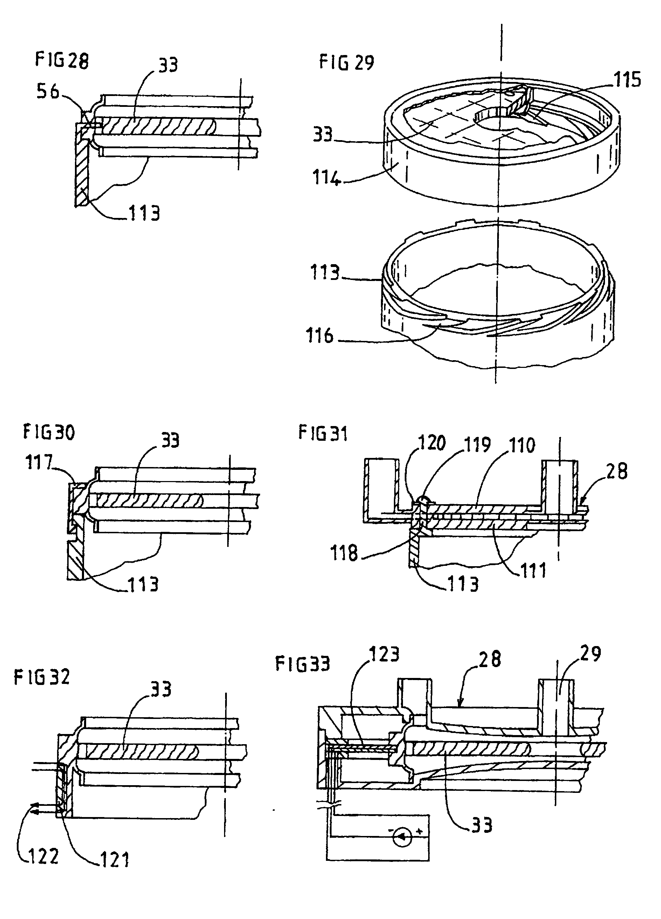

Par la figure 28 on a isolée la représentation de la liaison mécanique simple et économique entre la membrane 33 et un élément mobile 113 du moteur d'excitation qui assure l'application des forces motrices d'excitation 34 sur la membrane. L'élément mobile 113 se prolonge comme dans la figure 4 par une collerette rigide 56 et perforée qui pénètre dans la membrane 33 où elle est maintenue fixée par collage ou surmoulage. L'élément mobile 113 peut être le support d'une bobine ou le support d'une armature en matériau magnétique.

A la figure 29 on a illustré un autre mode de liaison de la membrane 33 à l'élément 113 appartenant à l'organe d'excitation. La membrane 33 est encastrée par sa périphérie dans une bague 114, laquelle est pourvue de filets 115 permettant, par vissage sur l'élément 113 au moyen de filets complémentaires 116, d'y être attelée. D'autres attelages, notamment du type baïonnette, peuvent convenir.

La figure 30 illustre un autre attelage de la membrane 33 à la pièce mobile 113 au moyen d'un collier extérieur 117 de manière que la membrane se trouve bridée en périphérie sur le bord supérieur de l'élément mobile 113.

Le mode de liaison selon la figure 31 concerne les variantes de corps de pompe décrits à la figure 27. L'élément mobile 113 de l'excitateur possède à son bord supérieur des plots 118 qui traversent le corps de pompe 28 formé par les deux membranes associées 110, 111 et comportent à leur extrémité une excroissance 119 coopérant avec un clip 120 qui emprisonne et pince la membrane.

L'exemple de réalisation de la figure 32 concerne une membrane 33 qui à sa périphérie possède en une seule pièce un élément 121 surmoulé, par exemple une bobine ou une armature magnétique, cet élément 121 est susceptible d'être sensible à un champ magnétique 122 généré par le moteur d'excitation. Cette disposition a pour avantage un prix de fabrication réduit puisqu'on élimine le support de cet élément sensible au champs magnétiques.

A la figure 33, la membrane 33 est attelée à un élément 123 qui constitue un moteur d'excitation piézo-électrique particulièrement adapté aux micropompes.

Pour obtenir une variation de la tension dans la membrane déformable mise en oeuvre dans les circulateurs de l'invention, on pourra incorporer aux extrémités de cette membrane, qu'elle soit plane, tubulaire ou circulaire, des éléments sensibles à un champ magnétique qui, correctement orienté, permettront de faire varier cette tension et ainsi la fréquence propre de la membrane. Ceci offre des possibilités de modulation de la puissance hydraulique du circulateur sans accroître la course de l'excitateur et l'amplitude des ondes.

Par exemple, dans la membrane illustrée par la figure 32, les éléments 121 incluent une bobine ou un matériau sensible aux champs magnétiques engendré par un organe électromagnétique de traction, c'est-à-dire en direction radiale, cet organe étant soit porté par le moteur d'excitation, soit par le corps de pompe lui-même.

On indiquera que les différentes géométries illustrées par les figures 1, 2 et 3 des circulateurs selon l'invention sont adaptées à des applications particulières. Ainsi, pour ce qui concerne la géométrie de la figure 1, ce circulateur trouve une application plus particulière dans les dispositifs de propulsion devant délivrer un fort débit sous un faible gradient de pression. La géométrie illustrée par la figure 2 concerne plus spécialement des pompes de faible encombrement et de faible débit. Pour ce qui concerne la figure 3, de géométrie circulaire, les circulateurs s'adressent au plus grand nombre d'applications avec des gradients de pression disponibles plus importants.

Les diverses dispositions décrites ci-dessus peuvent concourir à la réalisation de cassettes rigides qui d'une part incorporent les corps de pompes et d'autre part peuvent assurer de nombreuses fonctions supplémentaires notamment en ce qui concerne la connexion de la pompe avec les circuits hydrauliques en amont et en aval, les fonctions de prises de pression, de prises de débit, des fonction de filtration, de dégazage, de traitement du fluide et des fonctions d'addition de produit du genre médicament, traceur, produit d'analyse... Ces cassettes rigides trouvent alors des applications dans la réalisation de cartouches de dialyse, de cartouches de circulation extra-corporelle avec oxygénation du sang ou de cartouches d'injection d'insuline.

## Revendications

1. Circulateur de fluide à membrane comprenant un circuit hydraulique interne composé successivement d'un orifice d'admission (1, 15, 27), d'un corps de pompe (2, 16, 28) et d'un orifice de refoulement (3, 17, 29), le corps de pompe (2, 16, 28) délimitant en fonctionnement un espace (4, 18, 30) à parois rigides (5, 6, 7, 8, 19, 20, 31, 32) entre lesquelles est placée une membrane déformable (9, 21, 33) associée à des moyens (85, 86, 121) de création d'une tension parallèle à la circulation du fluide, pour constituer le support d'ondes se déplaçant de l'extrémité soumise à la force d'excitation vers son autre extrémité située du côté de l'orifice de refoulement, et comportant des moyens pour son attelage par son extrémité (11, 23, 35) du côté de l'orifice d'admission à un organe moteur (39) engendrant une force d'excitation périodique sensiblement normale à sa surface, **caractérisé en ce que** l'écartement des parois rigides est décroissant depuis l'orifice d'admission jusqu'à l'orifice de refoulement pour créer un amortissement forcé des ondes au cours de leur déplacement le long de la membrane, afin de créer un transfert d'énergie entre la membrane et le fluide se traduisant sous la forme d'un gradient de pression et d'un débit de fluide.

2. Circulateur de fluide selon la revendication 1, **caractérisé en ce que** la fréquence de l'excitation périodique est égale à l'une des fréquences propres de la membrane déformable (9, 21, 33) sous tension.

3. Circulateur de fluide selon l'une des revendications 1 et 2, **caractérisé en ce que** l'espace à parois rigides est un espace tubulaire plat (4), de largeur déterminée, dans lequel est située la membrane déformable plane (9) de même largeur que l'espace (4).

4. Circulateur de fluide selon la revendication 1 ou la revendication 2, **caractérisé en ce que** l'espace de circulation (18) est limité par deux parois rigides, de révolution et coaxiales (19, 20) entre lesquelles est située la membrane déformable (21) de forme tubulaire.

5. Circulateur de fluide selon l'une des revendications 1 et 2, **caractérisé en ce que** l'espace (30) à parois rigides est délimité par deux parois coaxiales en forme de disque (31, 32) entre lesquelles est placée une membrane déformable (33) également en forme de disque.

6. Circulateur de fluide selon l'une des revendications précédentes, **caractérisé en ce que** la membrane est un corps composite obtenu par l'assemblage de plusieurs parties de caractéristiques mécaniques différentes.

7. Circulateur de fluide selon l'une des revendications précédentes, **caractérisé en ce que** le circuit hydraulique comporte entre la membrane (33) et le corps de pompe (28) des reliefs agissant sur les caractéristiques de l'écoulement du fluide.

8. Circulateur de fluide selon l'une des revendications précédentes, **caractérisé en ce que** le corps de pompe (28) forme une unité intégrant de manière inséparable la membrane (33), les orifices d'admission et de refoulement (27, 28) et les parois de l'espace (30) dans lequel est logée la membrane, ce corps de pompe étant séparable du circulateur.

9. Circulateur de fluide selon l'une des revendications précédentes, **caractérisé en ce qu'**il comporte un moteur électromagnétique (39) engendrant une force d'excitation périodique et alimenté par un courant alternatif d'excitation dont l'intensité modulée permet une modulation de la force d'excitation et donc de la puissance hydraulique fournie par le circulateur.

10. Circulateur de fluide selon l'une des revendications précédentes **caractérisé en ce qu'**il comporte au moins un organe de traction électromagnétique engendrant des forces (12, 13, 24, 25, 36 et 37) appliquées aux extrémités de la membrane concourant à sa tension dans la direction de propagation des ondes issues de son excitation périodique.

## Patentansprüche

1. Fluid-Umwälzpumpe mit Membran, umfassend einen inneren Hydraulikkreislauf, der aufeinanderfolgend aus einer Einlaßöffnung (1, 15, 27), einem Pumpengehäuse (2, 16, 28) und einer Auslaßöffnung (3, 17, 29) besteht, wobei das Pumpengehäuse (2, 16, 28) im Betrieb einen Raum (4, 18, 30) mit starren Wänden (5, 6, 7, 8, 19, 20, 31, 32) begrenzt, zwischen denen eine verformbare Membran (9, 21, 33) angeordnet ist, die mit Mitteln (85, 86, 121) zum Erzeugen einer zur Strömung des Fluids parallelen Spannung verbunden ist, um den Träger für Wellen zu bilden, die von dem Ende, das der Erregungskraft ausgesetzt ist, zu ihrem anderen Ende laufen, das sich auf der Seite der Auslaßöffnung befindet, und mit Mitteln für ihr Ankoppeln über ihr auf der Seite der Einlaßöffnung befindliches Ende (11, 23, 35) an ein Antriebsorgan (39), das eine periodische Erregungskraft erzeugt, die im wesentlichen senkrecht zu ihrer Oberfläche gerichtet ist, **dadurch gekennzeichnet, daß** der Abstand zwischen den starren Wänden von der Einlaßöffnung bis zur Auslaßöffnung abnimmt, um eine erzwungene Dämpfung der Wellen im Laufe ihrer Ausbreitung entlang der Membran zu erzeugen und damit eine Energieübertragung zwischen der Membran und dem Fluid zu verursachen, die sich in Form eines Druckgradienten und eines Ausstoßes von Fluid äußert.

2. Fluid-Umwälzpumpe nach Anspruch 1, **dadurch gekennzeichnet, daß** die Frequenz der periodischen Erregung gleich einer der Eigenfrequenzen der gespannten verformbaren Membran (9, 21, 33) ist.

3. Fluid-Umwälzpumpe nach einem der Ansprüche 1 und 2, **dadurch gekennzeichnet, daß** der Raum mit starren Wänden ein flacher, röhrenförmiger Raum (4) mit vorgegebener Breite ist, in dem sich die flache verformbare Membran (9) mit einer Breite befindet, die gleich der des Raumes (4) ist.

4. Fluid-Umwälzpumpe nach Anspruch 1 oder Anspruch 2, **dadurch gekennzeichnet, daß** der Zirkulationsraum (18) von zwei rotationssymmetrischen und koaxialen starren Wänden (19, 20) begrenzt ist, zwischen denen sich die röhrenförmige verformbare Membran (21) befindet.

5. Fluid-Umwälzpumpe nach einem der Ansprüche 1 und 2, **dadurch gekennzeichnet, daß** der Raum (30) mit starren Wänden von zwei koaxialen scheibenförmigen Wänden (31, 32) begrenzt ist, zwischen denen eine ebenfalls scheibenförmige verformbare Membran (33) angeordnet ist.

6. Fluid-Umwälzpumpe nach einem der vorhergehenden Ansprüche, **dadurch gekennzeichnet, daß** die Membran ein Verbundkörper ist, der durch Montage mehrerer Teile mit unterschiedlichen mechanischen Eigenschaften erhalten wird.

7. Fluid-Umwälzpumpe nach einem der vorhergehenden Ansprüche, **dadurch gekennzeichnet, daß** der Hydraulikkreislauf zwischen der Membran (33) und dem Pumpengehäuse (28) Reliefs hat, die die Fließeigenschaften des Fluids beeinflussen.

8. Fluid-Umwälzpumpe nach einem der vorhergehenden Ansprüche, **dadurch gekennzeichnet, daß** das Pumpengehäuse (28) eine Einheit bildet, welche die Membran (33), die Ein- und Auslaßöffnungen (27, 29) und die Wände des Raumes (30), in dem die Membran untergebracht ist, auf unlösbare Weise integriert, wobei dieses Pumpengehäuse von der Umwälzpumpe getrennt werden kann.

9. Fluid-Umwälzpumpe nach einem der vorhergehenden Ansprüche, **dadurch gekennzeichnet, daß** sie einen elektromagnetischen Antrieb (39) enthält, der eine periodische Erregungskraft erzeugt und von einem Erregungsstrom in Form eines Wechselstroms gespeist wird, dessen modulierte Intensität eine Modulation der Erregungskraft und folglich der von der Umwälzpumpe gelieferten hydraulischen Leistung ermöglicht.

10. Fluid-Umwälzpumpe nach einem der vorhergehenden Ansprüche, **dadurch gekennzeichnet, daß** sie mindestens ein elektromagnetisches Zugorgan enthält, das Kräfte (12, 13, 24, 25, 36 und 37) erzeugt, die auf die Enden der Membran ausgeübt werden und zu deren Spannung in Ausbreitungsrichtung der Wellen beitragen, die aufgrund ihrer periodischen Erregung ausgesandt werden.

## Claims

1. A vibrating membrane fluid circulator comprising an internal hydraulic circuit made up in succession of an admission orifice (1, 15, 27), a pump body (2, 16, 28), and a delivery orifice (3, 17, 29), the pump body (2, 16, 28) defining, in operation, a space (4, 18, 30) having rigid walls (5, 6, 7, 8, 19, 20, 31, 32) between which a deformable membrane (9, 21, 33) is placed in cooperation with means to create a tension parallel to the fluid circulation in order to constitute the medium for waves travelling from the end subjected to the excitation force towards its opposite end situated adjacent to the delivery orifice and having means for coupling its end (11, 23, 35) adjacent to the admission orifice to a motor member (39) generating a periodic excitation force substantially normally to the surface thereof **characterized in that** the spacing of the rigid walls is decreasing going away from the admission orifice towards the delivery orifice to generate a forced damping of the waves during their travel along the membrane, so as to transfer energy from the membrane to the fluid giving rise to a pressure gradient and to a fluid flow.

2. A fluid circulator according to claim 1, **characterized in that** the periodic excitation frequency is equal to one of the natural frequencies of the mechanical system constituted by the deformable membrane (9, 21, 33) under tension.

3. A fluid circulator according to claim 1 or 2, **characterized in that** the space with rigid walls is a flat tubular space (4), of determined width, in which there is situated the deformable membrane (9) which is plane and of the same width as the space (4).

4. A fluid circulator according to claim 1 or claim 2, **characterized in that** the circulation space (18) is defined by two rigid walls that are circularly symmetrical and coaxial (19, 20), with the deformable membrane (21) being situated between them and being tubular in shape.

5. A fluid circulator according to claim 1 or 2, **characterized in that** the space (30) having rigid walls is defined by two disk-shaped walls (31, 32) on a common axis with a deformable membrane (33) being placed between them, which membrane is likewise disk-shaped.

6. A fluid circulator according to any preceding claim, **characterized in that** the membrane is a composite body obtained by assembling together a plurality of portions having different mechanical characteristics.

7. A fluid circulator according to any preceding claim, **characterized in that** the hydraulic circuit includes relief between the membrane (33) and the pump body (28), which relief acts on the flow characteristics of the fluid.

8. A fluid circulator according to any preceding claim, **characterized in that** the pump body (28) forms a unit integrating the membrane (33), the admission and delivery orifices (27, 28), and the walls of the space (30) in which the membrane is housed in inseparable manner, said pump body itself being separable from the circulator.

9. A fluid circulator according to any preceding claim, **characterized in that** it comprises an electromagnetic motor (39) generating a periodic excitation force and fed with an alternating excitation current of intensity which is modulated to modulate the excitation force and thus the hydraulic power delivered by the circulator.

10. A fluid circulator according to any preceding claim, **characterized in that** it includes at least one electromagnetic traction member generating forces (12, 13, 24, 25, 36 and 37) applied to the ends of the membrane in addition to the tension it has in the propagation direction of the waves generated by its periodic excitation.
